# EUROPEAN PATENT SPECIFICATION

(11) **EP 0 719 545 B1**
(45) Date of publication and mention of the grant of the patent: **05.06.2002**
(21) Application number: 96200145.9
(22) Date of filing: 05.07.1991
(51) Int. Cl.: A61K 9/00, A61K 9/06, A61K 47/10, A61K 47/18, A61K 47/34, A61K 47/36

(54) **Thermally reversible and irreversible gels**
Thermisch umkehrbare und nichtumkehrbare Gele
Gels thermiquement réversibles et irréversibles

(30) Priority: 07.08.1990 US 563638; 07.08.1990 US 563639; 07.08.1990 US 563764; 07.08.1990 US 563640; 26.10.1990 US 604701; 26.10.1990 US 604705
(43) Date of publication of application: 03.07.1996
(62) Divisional of application: 91306120.6
(73) Proprietor: MDV Technologies, Inc., Dearborn, Michigan 48120 (US)
(72) Inventor: Viegas, Tracey X., Hoover, Alabama 35244 (US); Reeve, Lorraine E., Ann Arbor, MI 48106 (US); Henry, Raymond L., Grosse Pointe woods, Michigan 48236 (US)
(74) Representative: W.P. Thompson & Co.

(56) References cited:
- EP-A- 0 126 684
- EP-A- 0 227 494
- EP-A- 0 243 930
- EP-A- 0 455 396
- US-A- 4 188 373

## Description

The present invention relates to ophthalmic drug delivery systems, ophthalmic corneal protective devices, and a surgical device for correction of corneal astigmatism, myopia, and hyperopia comprising an aqueous gel.

Over the years, methods have been developed to achieve the efficient delivery of a therapeutic drug to a mammalian body part requiring pharmaceutical treatment. Use of an aqueous liquid which can be applied at room temperature as a liquid but which forms a semisolid gel when warmed to body temperature has been utilized as a vehicle for drug delivery since such a system combines ease of application with greater retention at the site requiring treatment than would be the case if the aqueous composition were not converted to a gel as it is warmed to mammalian body temperature. In the U.S. Patent No. 4,188,373, PLURONIC® polyols are used in aqueous compositions to provide thermally gelling aqueous systems. Adjusting the concentration of the polymer provides the desired sol-gel transition temperature, that is, the lower the concentration of polymer, the higher the sol-gel transition temperature, after crossing a critical concentration minimum, below which a gel will not form.

In U.S. 4,474,751; '752; '753; and 4,478,822 drug delivery systems are described which utilize thermosetting gels; the unique feature of these systems is that both the gel transition temperature and/or the rigidity of the gel can be modified by adjustment of the pH and/or the ionic strength, as well as by the concentration of the polymer.

Other patents disclosing pharmaceutical compositions which rely upon an aqueous gel composition as a vehicle for the application of the drug are U.S. Patent Nos. 4,883,660; 4,767,619; 4,511,563; and 4,861,760. Thermosetting gel systems are also disclosed for application to injured mammalian tissues of the thoracic or peritoneal cavities in U.S. 4,911,926.

Ionic polysaccharides have been used in the application of drugs by controlled release. Such ionic polysaccharides as chitosan or sodium alginate are disclosed as useful in providing spherical agglomerates of water-insoluble drugs in the Journal of Pharmaceutical Sciences volume 78, number 11, November 1989, Bodmeier et al. Alginates have also been used as a depot substance in active immunization, as disclosed in the Journal of Pathology and Bacteriology volume 77, (1959), C.R. Amies. Calcium alginate gel formulations have also found use as a matrix material for the controlled release of herbicides, as disclosed in the Journal of Controlled Release, 3 (1986) pages 229-233, Pfister et al.

In U.S. 3,640,741, a molded plastic mass composed of the reaction product of a hydrophilic colloid and a cross-linking agent such as a liquid polyol, also containing an organic liquid medium such as glycerin, is disclosed as useful in the controlled release of medication or other additives. The hydrophilic colloid can be carboxymethyl cellulose gum or a natural alginate gum which is cross-linked with a polyol. The cross-linking reaction is accelerated in the presence of aluminum and calcium salts. In U.S. 4,861,760 and 4,895,724, compositions are disclosed for the controlled release of pharmacological macromolecular compounds contained in a matrix of a polysaccharide.

In U.S. 4,795,642, there are disclosed gelatin-encapsulated, controlled-release compositions for release of pharmaceutical compositions, wherein the gelatin encloses a solid matrix formed by the cation-assisted gellation of a liquid filling composition incorporating a vegetable gum togetner with a pharmaceutically-active compound. The vegetable gums are disclosed as polysaccharide gums such as alginates which can be gelled utilizing a cationic gelling agent such as an alkaline earth metal cation.

Corneal protective devices are needed in cases in which corneal injury occurs and the immobilization of the eye using an eye patch is not resorted to. Molded collagen shields nave been developed for this use. These are often not satisfactory because they lack sufficient flexibility to conform to the individual corneal curvature. The clinical uses of collagen shields are disclosed by Poland et al. in Journal of Cataract Refractive Surgery, Volume 14, September 1988, pages 489-491. The author describes the use of collagen shields immersed in tobramycin solution in order to rehydrate the collagen prior to use. These are described as useful following cataract extraction or in patients having nonsurgical epithelial healing problems. More rapid healing of epithelial defects after surgery resulted from the use of the collagen shield. Collagen shields have also been utilized as agents for the delivery of drugs to the cornea as disclosed in Reidy et al Cornea, in press, 1989 the Raven Press, Ltd., new York and Shofner et al, Opthalmology Clinics of North America, Vol. 2, No. 1, March 1989, pages 15-23.

Refractive surgery has been promoted in the United States and Russia over the past few years but its acceptance has been limited because of the poor predictability of the final optical results which include a resulting glare from incisions that encroach upon the optical zone. Techniques that rely upon the surgical production of corneal incisions have yielded inconsistent results because these surgical incisions in the cornea have been found to vary considerably in depth and length.

Laser keratectomy has been shown to be capable of yielding a more accurately controlled depth of corneal excision since each individual laser pulse excises a specific amount (0.2 to 10.0 mm) of corneal tissue. Accordingly, the depth of excised tissue is in theory uniform and predictable, provided that the energy distribution is homegeneous across the laser beam. Since the primary locus of astigmatism is in the cornea, surgical intervention for astigmatism is more important than for the correction of other refractive errors, especially since spectacle or contact lens correction is of limited value in compensating for large astigmatic errors.

Tne excimer laser was introduced to opthalmology in 1983 (Trokel, S., et al., "Excimer surgery of the cornea," Am. J. Ophthalmol. 96: 710-715, 1983). The depth of incision with short intense pulses permitted great precision to be achieved in tests on freshly enucleated cow eyes. The photochemical laser-tissue interaction is not thermal, permitting direct breaks of organic molecular bonds without involving optical breakdown in adjacent tissue. Early experimental results in rabbits revealed problems of (1) corneal stromal swelling, probably in response to disturbed water relationships due to compromise of the epithelial barrier and severing of the lamellae and (2) rearrangement of endothelial cells resulting from loss of contact inhibition (Marshall, J., et al., "An ultrastructural study of corneal incisions induced by an excimer laser at 193 nm", Othalmology 92: 749-758, 1985). Experiments with freshly enucleated human eyes indicated that flattening obtained by excimer laser ablation correlated with results of clinical scalpel radial keratotomy, but evaluation of the effects on wound healing and possible damage to adjacent structures was not addressed (Cotliar, A.M., et al., "Excimer laser radial keratotomy," Opthalmology 92: 206-208, 1985). It was, however, suggested that this laser may become very useful in applications including penetrating and lamellar keratoplasty, keratomileusis, and epikeratophakia. Control of the area and depth of pulses using photolithographed masks resulted in ability to produce narrow cuts (20 um) and at depths depending on pulse number (Puliafito, C.A., et al., "Excimer laser ablation of the cornea and lens", Ophthalmology 92: 741-748, 1985). These controlled ablations had only very narrow bands of destruction at the adjacent edges. These studies led to the quantitation of laser ablation (Kruegar, R.R. and S.L. Trokel, "Quantitation of corneal ablation by ultraviolet laser light", Arch. Opthalmol. 103: 1741-1742, 1985). Excimer far UV radiation can be controlled to produce minimal adjacent tissue damage providing the angle and depth can be precisely controlled.

EP 455 396 (A54.3 cite) discloses an antibacteria formulation comprising xanthan gum and poloxamer 407 (BASF).

EP 126 684 discloses a drug delivery system utilizing thermosetting gel.

EP 227 494 discloses a composition of the type which undergoes liquid - gel phase transition. The composition contains at least one polysaccharide.

Review of the literature clearly reveals that far UV vaporization (ablation with an excimer laser at 193 nm, far example) is a feasible means to sculpture or reprofile the cornea to correct nearsightedness, farsightedness, astignatism, corneal scars, corneal densities, etc. The healing appears to parallel or to be equal to healing after scalpel intervention, providing the proper guidelines for pusling and duration are followed. There remains a need to control the contour and uniformity of the ablated surface. Such control will reduce the adverse structural and biological response of the cornea and insure that a desired corrective change results.

The use of a mask of nearly identical optical density to the cornea that can be preformed on the surface of the cornea so as to provide a smooth surface of exact contour would solve many of the problems still remaining which thus far have prevented precise control of laser beam keratotomy. Such a mask would be required to withstand exposure to moist gases directed tangentally to the corneal surface throughout the duration of exposure to the laser to remove ablated debris. The modulation of the beam energy distribution of the laser in a controlled fashion should also be provided by such a corneal mask. The use of a smooth ablatable mask having a known contour and having the density of the cornea would aid in insuring accurate direction and depth of a tangental cut utilizing a laser beam. The ablatable mask of the invention provides such advantages.

In accordance with the present invention there is provided a pH buffered thermally-reversible, aqueous gel composition characterized in that it comprises a polyoxyalkylene block copolymer and an alginate or chitosan ionic polysaccharide.

In one embodiment of the invention pharmaceutical compositions are disclosed generally containing pharmacologically active medicaments which are useful in providing treatments to areas of the mammalian body requiring the application of a medicament or diagnostic agent. In addition, the compositions of the invention are useful, without the inclusion of a medicament, as a protective corneal shield or ablatable corneal mask in a laser keratectomy process. The compositions of the invention provide a physiologically acceptable media having a buffered pH and an osmotically balanced vehicle, generally characterized as hyper-osmotic, iso-osmotic, or hypo-osmotic. Preferably, an isotonic mixture is provided which is iso-osmotic with body fluids and has a buffered pH similar to bodily fluids, such as lacrimal tears. The pH and osmotic pressure of lacrimal tears is about pH 7.4 and 290 mOsm/kg. In addition, the pharmaceutical compositions are, optionally, sterilized so as to insure that the compositions of the invention do not provide a source of infection.

The compositions of the invention in one embodiment comprise aqueous mixtures of a polyoxyalkylene polymer and an ionic polysaccharide, optionally containing a latent form of a counter-ion to gel the polysaccharide upon release of the counter-ion and to render the gelled mixture thermally irreversible so that it remains a gel upon cooling. The counter-ion can be microencapsulated in a heat sensitive medium, for instance, the walls of the microcapsule can be made of mono-, di-, or tri-glycerides or other natural or synthetic heat sensitive polymer medium. Alternatively, ion exchange resins can be incorporated in the compositions of the invention so as to release the desired counter-ion upon contact with an environment opposite in pH to the pH of the ion exchange resin. The aqueous mixture can be delivered to the ophthalmic area of the mammalian body requiring treatment as a low viscosity liquid at ambient temperatures which, upon contact witn the higher temperature mammalian body, forms a semi-solid gel having a very high viscosity. Release of the counter-ion further strengthens the gel and renders it irreversible upon cooling. Alternatively, a two part system can be used in which the counter-ion can be separately applied to the semi-solid gel formed by the polyoxyalkylene polymer upon contact with tne mammalian body. This further strengthens the gel and renders it irreversible upon cooling. Because the preferred pharmaceutical compositions of the invention are low viscosity liquids at ambient temperatures, they easily pass to various body areas insuring maximum contact between exposed tissue and the pharmaceutical composition of the invention. The pharmaceutical gel compositions of the invention can be either peeled away or allowed to be absorbed over time. The gels are gradually weakened upon exposure to mammalian body pH conditions.

In a preferred embodiment, the compositions of the invention can be delivered to the area of the mammalian body requiring treatment as a low viscosity liquid at ambient temperatures which, upon contact with the mammalian body, forms a semi-solid gel having a very high viscosity. Because the preferred pharmaceutical compositions of the invention are low viscosity liquids at ambient temperatures, they easily pass to various ophthalmic areas insuring maximum contact between exposed tissue and the pharmaceutical composition of the invention. If necessary, the preferred pharmaceutical compositions of the invention can be washed away under a gentle stream of cool water, thus minimizing the risk of further injury and pain to the patient upon removal.

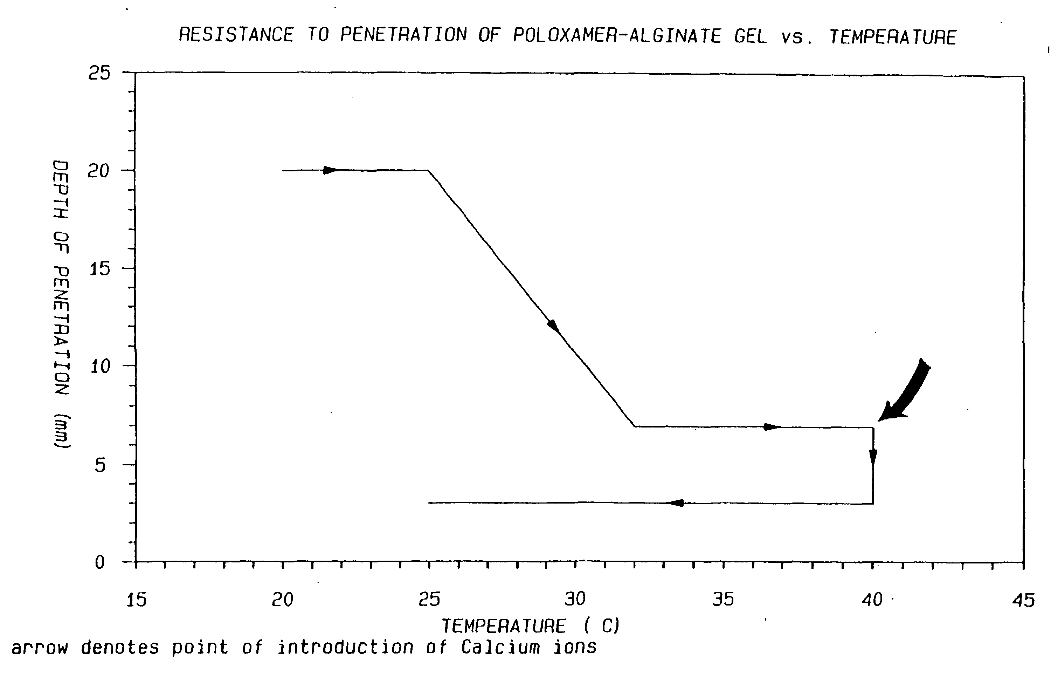

The invention will be further described, by way of example only, with reference to the drawing.

The drawing provides a curve showing the penetration, as measured by a Precision Universal Penetrometer, of a 20mm thickness aqueous gel comprising poloxamer 407 and an alginate prepared in accordance with the procedure of Example 1. The scale at the left side of the plot indicates the depth of penetration, while the scale on the bottom of the plot indicates the temperature of the composition wnen tested. The arrow in the plot indicates the point at which an aqueous solution of calcium ions at a concentration of 0.137 molar is made to contact the gelled poloxamer 407/alginate solution so as to render thermally-irreversible the gelled mixture and prevent it from becoming fluid at ambient temperature.

It has been found that aqueous pharmaceutical vehicles containing a polyoxyalkylene block copolymer, which have the unique feature, in a preferred embodiment, of being liquid at ambient temperatures and transitioning at mammalian body temperatures to a semi-solid gel, can be rendered thermally-irreversible (no longer a liquid at ambient temperature) and resistant to shear thinning. Upon contacting the mixture with a counter-ion, the polyoxyalkylene polymer aqueous gel becomes more resistant to penetration by the inclusion of a polysaccharide in admixture with the polyoxyalkylene polymer. The venicles are, preferably, made isotonic or iso-osmotic in the gel state and can be buffered to the pH of mammalian body fluids, such as lacrimal tears. The pH and osmotic pressure of such bodily fluids are 7.4 and 290 mOsm/kg, respectively. It is, accordingly, advantageous to deliver a pharmacologically active medicament in gel form to an area of the mammalian body requiring pharmacological treatment under pH and osmotic pressure conditions which match those of bodily fluids. Optionally, the pharmaceutical compositions of the invention can be provided in a sterile condition.

A wide variety of polyoxyalkylene polymers are suitable for the preparation of the pharmaceutical compositions of the invention. Generally, it is necessary to adjust the polymer concentration in aqueous solution so as to obtain the desired sol-gel transition temperature in order that the compositions can be provided as low viscosity liquids at ambient temperature, yet form semi-solid gels at mammalian body temperatures. In addition to the concentration of the polymer and the concentration of a water soluble or dispersible pharmacologically active medicament, other suitable excipients must be added so as to provide the preferred isotonic, iso-osmotic properties.

In addition to those well-known polyoxyalkylene block copolymers described below which are suitable in the formation of the pharmaceutical compositions of the invention, other less well-known polyoxyalkylene polymers which form gels at low concentrations in water, are suitable. One such polymer is described in U.S. Patent 4,810,503, These polymers are prepared by capping conventional polyether polyols with an alpha-olefin epoxide having an average of 20 to 45 carbon atoms, or mixtures thereof.
Aqueous solutions of these polymers gel in combination with surfactants, which can be ionic or nonionic. The combination of the capped polyether polymers and the surfactants provide aqueous gels at low concentrations of the capped polymer and surfactants, which generally do not exceed 10% by weight total. Detailed methods of preparing these aqueous gels are disclosed in U.S. 4,810,503. Preparation of said aqueous gels is generally described below. Preferred surfactants for use in preparing these gels are also disclosed in said patent.

A conventional copolymer polyether polyol is prepared by preparing block or heteric intermediate polymers of ethylene oxide and at least one lower alkylene oxide having 3 to 4 carbon atoms as intermediates. These are then capped with the alpha-olefin epoxide to prepare the polymers. Ethylene oxide homopolymers capped with said alpha-olefin oxides are also useful as intermediates.

The heteric copolymer intermediate is prepared by mixing ethylene oxide and at least one lower alkylene oxide having 3 to 4 carbon atoms with a low molecular weight active hydrogen-containing compound initiator having at least two active hydrogens and preferably, 2 to 6 active hydrogen atoms such as a polyhydric alcohol, containing from 2 to 10 carbon atoms and from 2 to 6 hydroxyl groups, heating said mixture to a temperature in the range of 50° C. to 150° C., preferably from 80° C. to 130° C., under an inert gas pressure preferably from (30 psig to 90 psig) 206.8 x 10³ to 620.5 x 10³Nm⁻².

A block copolymer intermediate is prepared by reacting either the ethylene oxide or said alkylene oxide having 3 to 4 carbon atoms with said active hydrogen-containing compound followed by reaction with the other alkylene oxide.

The ethylene oxide and the alkylene oxides having from 3 to 4 carbon atoms are used in said intermediates in amounts so that the resulting polyether product will contain at least 10 percent by weight, preferably about 70 percent to about 90 percent by weight, ethylene oxide residue. The ethylene oxide homopolymer intermediate is prepared by reacting ethylene oxide with said active hydrogen-containing compound. The reaction conditions for preparing the block copolymer and ethylene coxide homopolymer intermediates are similar to those for the heteric copolymer intermediate. The temperature and pressure are maintained in the above ranges for a period of about one hour to ten hours, preferably one to three hours.

The alpha-olefin oxides which are utilized to modify the conventional polyether intermediate of the prior art are those oxides and the commercially available mixtures thereof generally containing an average of about 20 to 45, preferably about 20 to 30, carbon atoms. The amount of alpha-olefin required to obtain the more efficient capped polyethers is generally about 0.3 to 10 percent, preferably about 4 to 8 percent, of the total weight of the polyethers.

Since the preparation of heteric and block copolymers of alkylene oxides and ethylene oxide homopolymers are well known in the art, further description of the preparation of said polymers is unnecessary. Further details of the preparation of heteric copolymers of lower alkylene oxide can be obtained in U.S. Pat. No. 3,829,506.

Further information on the preparation of block copolymers of lower alkylene oxides can be obtained in U.S. Pat nos. 3,535,307; 3,036,118; 2,979,578; 2,677,700; and 2,675,619.

The surfactants may be ionic or non-ionic and many surfactants and types of surfactants may be employed. While all surfactants may not be effective in the preparation of the isotonic gels of the instant invention, the fact that many are effective makes it a simple matter for one skilled in the art to select such surfactants with a minimum of trial and error.

The amounts of capped polyether polymer and surfactant used in the aqueous compositions of the invention may be as little as 1.0 percent by weight or less of each depending on the type and amount of the other component. There appears to be no maximum amount of either component than that dictated by economic considerations. However, the total amount of capped polymer and surfactant would generally not exceed 10 percent by weight.

The ionic polysaccharides found useful in the present invention are hydrophilic colloidal materials and include the natural gums such as alginate gums, i.e., the ammonium and alkali metal salts of alginic acid and mixtures thereof as well as chitosan, which is a common name for the deacetylated form of chitin. Chitin is a natural product comprising poly-(N-acetyl-D-glucosamine). The alginates are available as dry powders from Protan, Inc., Commack, New York and from Kelco Company, San Diego, California.

The ionic polysaccharides are natural polymers such as chitosan or alginates. Aqueous solutions of alginate ionic polysaccharides form gels upon contact with aqueous solutions of counter-ions such as calcium, strontium, aluminum, etc. Aqueous solutions of chitosan form gels upon contact with a metal tripolyphosphate counter-ion. The discovery forming the basis of this application is that when ionic polysaccharides are present in aqueous solutions in admixture with certain polyoxyalkylene block copolymers and a counter-ion, that such mixtures form thermally-irreversible gels instead of the thermo-reversible gels known to form with aqueous solutions of certain polyoxyalkylene block copolymers.

Generally, the alginates can be any of the water-soluble alginates including the alkali metal alginates, such as sodium, potassium, lithium, rubidium and caesium salts of alginic acid, as well as the ammonium salt, as well as the soluble alginates of an organic base such as mono-, di-, or tri-ethanolamine, and aniline.

Generally. 0.2% to 2.5% by weight and, preferably, 0.5% to 1.5% by weight of alginate or chitosan ionic polysaccharides, based upon the total weight of the composition, are used to obtain the thermo-irreversible compositions of the invention. In general, the drug delivery composition of the invention will contain 0.01% to 60% by weight of medicament or pharmaceutical, 10% to 50% by weight of the polyoxyalkylene polymer, and 80% to 20% by weight of water together with the above amounts of ionic polysaccharide. In special situations, these amounts may be varied to increase or decrease the dosage or gel properties.

Useful counter-ions for thermo-irreversibly gelling the alginate ionic polysaccharide in combination with the polyoxyalkylene polymer compositions of the invention are cationic gelling agents, preferably, comprising a divalent or trivalent cation. Useful divalent cations include the alkaline earth metals, preferably, selected from the group consisting of calcium and strontium. Useful trivalent cations include aluminum. The most preferred counter-ions for gelling an alginate are contained in ionic compounds selected from pharmaceutically-acceptable gluconates, flourides, citrates, phpsphates, tartrates, sulfates, acetates, borates, and chlorides having alkaline earth metal cations such as. calcium and strontium. Especially preferred counter-ion containing inorganic salts for use as ionic polysaccnaride gelling agents include such inorganic salts as the chloride salts, such as strontium chloride, calcium chloride, and mixtures thereof. Generally, a molar ratio of counter-ion to chitosan or alginate of 1:1 to 10:1, preferably, 2:1 to 5:1, and most preferably, 3:1 to 5:1 is used to render the compositions of the invention thermally-irreversibly gelled.

While the counter-ion, such as calcium or other counter-ions may be obtained by contact with bodily fluids, it is preferred that the counter-ion in latent form be added to the alginate ionic polysaccharide and polyoxyalkylene polymer compositions of the invention. Alternatively, a counter-ion can be added to the alginate ionic polysaccharide and polyoxyalkaline polymer compositions of the invention utilizing a two part system in which the counter-ion is applied to the remaining components of the drug delivery system subsequent to application. Incorporation of the counter-ion in a latent form together with the alginate ionic polysaccharide and polyoxyalkylene polymer compositions of the invention may be accomplished by either encapsulating an aqueous solution of the counter-ion gelling agents, previously described above or by the incorporation of the counter-ion gelling agent into a matrix which provides for the controlled, slow-release of the gelling agent. For instance, the gelatin-encapsulated controlled-release compositions disclosed in U.S. 4,795,642, disclose the preparation of a gelatin shell encapsulating a controlled-release formulation in which the gelatin composition includes calcium chloride as the gelling agent. Alternatively, the counter-ion can be incorporated as an aqueous solution of a cationic gelling agent encapsulated in a vesicle composed, for instance, of alpha-tocopherol, as disclosed in U.S. 4,861,580,

Generally, aqueous solutions of chitosan can be gelled with multivalent anion gelling agents, preferably, comprising a metal polyphosphate, such as an alkali metal or ammonium polyphosphates, pyrophosphates, or metaphosphates. Representative metaphosphate, pyrophosphate, and polyphosphate gelling agents include sodium and potassium, polyphosphates, sodium and potassium pyrophosphates, sodium and potassium metaphosphates, and sodium and ammonium (mono-, di-, tri-) phosphates.

Polyphase systems are also useful and may contain non-aqueous solutes, non-aqueous solvents, and other non-aqueous additives. Homogeneous, polyphase systems can contain such additives as water insoluble high molecular weight fatty acids and alcohols, fixed oils, volatile oils and waxes, mono-, di-, and triglycerides, and synthetic, water insoluble polymers without altering the functionality of the system.

With specific reference to the use of the pharmaceutical compositions as ophthalmic drug delivery compositions, it is noted that for the avoidance of adverse physiological effects to the eye, it is desirable that the pH and osmolality of the pharmaceutical vehicle be matched to the pH and osmolality of the eye. In addition, it is noted that a large percentage of drugs administered to the eye are lost as a result of lacrimal drainage. This applies especially in situations in which a liquid composition containing a pharmacologically active medicament is applied to the cornea of the eye. Accordingly, in such cases, only a small fraction of the pharmaceutical composition administered to the eye remains in contact with the cornea for a few minutes and an even smaller fraction penetrates into the cornea. To overcome these disadvantages, it is known to use viscous solutions, gels, ointments, or solid eye implants containing pharmacologically active medicaments. While progress has been made in the delivery of drugs by the use of solid implants, many patients find it difficult to tolerate the introduction of the implants into the conjunctival areas.

To solve this problem, drug delivery systems which are liquid at room temperature and assume a semi-solid form at human body temperature have been proposed, such as those described in U.S. Patent 4,188,373, which disclose the use of PLURONIC® polyols. In U.S. Patent 4,861,760 and U.S. Patent 4,474,751, ophthalmic drug delivery systems are disclosed which show liquid-gel phase transitions. In the '751 Patent, polymers are disclosed which are tetra substituted derivatives of ethylenediamine, propylenediamine, butylenediamine, pentylenediamine, or hexylenediamine. These are described as block copolymers of poly(oxypropylene) and poly(oxyethylene) of various chain lengths. These polymers are described for use in aqueous drug delivery vehicles, which contain from 10% to 50% by weight of copolymer based on the weight of the total drug delivery vehicle.

In the '760 Patent, referred to above, the liquid-gel phase transition compositions for ophthalmological use contain polymers which form gels at concentrations 10-100 fold lower than those used in systems such as the '751 Patent, involving thermogellation. Accordingly, the drug delivery systems of the '760 Patent are said to be very well tolerated by the eye. The polymers utilized in the drug delivery vehicles of the '760 Patent are described as polysaccharides obtained by fermentation of a microorganism.

Generally, the polyoxyalkylene block copolymers of the invention are defined as follows:
a polyoxyalkylene block copolymer of the formula 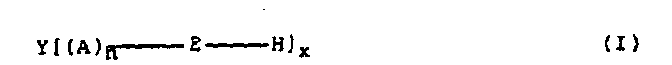 wherein A is a polyoxyalkylene moiety having an oxygen/carbon atom ratio of less than 0.5, x is at least 2, Y is derived from water or an organic compound containing x reactive hydrogen atoms, E is a polyoxyethylene moiety constituting at least 60% by weight of the copolymer, n has a value such that the average molecular weight is at least 500 to 900, as determined by the hydroxyl number of an intermediate, 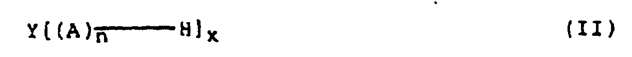 and the total average molecular weight of the copolymer is at least about 5000.

Preferred are polyoxyalkylene block copolymers of the formula:

HO(C₂H₄O)_{b}(C₄H₈O)ₐ(C₂H₄O)_{b}H (III)

wherein in III, a is an integer such that the hydrophobe base represented by (C₄H₈O)ₐ has a molecular weight of at least about 500 as determined by hydroxyl number, the polyoxyethylene chain constituting at least about 70% by weight of the copolymer, and the copolymer having a total average molecular weight of at least 15,000, or

HO(C₂H₄O)_{b}(C₃H₆O)ₐ(C₂H₄O)_{b}H (IV)

wherein in IV, a is an integer such that the hydrophobe base represented by (C₃H₆O)ₐ has a molecular weight of at least about 900 average molecular weight, as determined by hydroxyl number, the polyoxyethylene chain constitutes at least about 70% by weight of the copolymer, and the copolymer having a total average molecular weignt of at least about 15,000, or 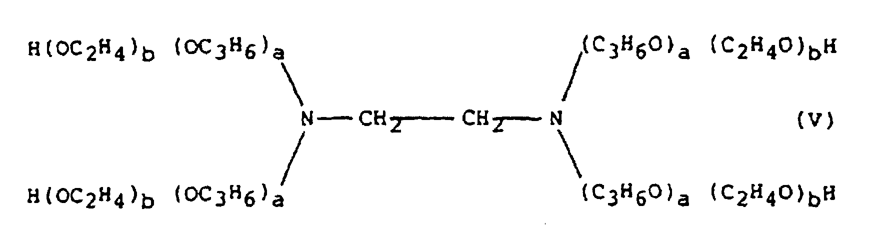 wherein in V, a and b are integers such that the copolymer has a hydrophobe molecular weight of at least about 1500, a hydrophile content of at least about 70%, and a total average molecular weight of at least about 15,000.

Most preferred are the polyoxyalkylene block copolymers of the formula: 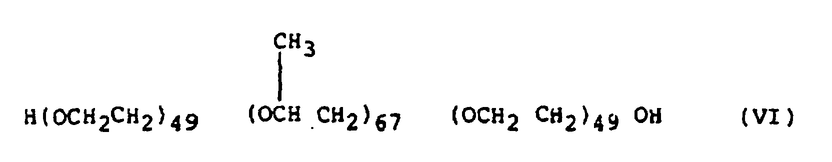 These polymers are present, preferably, in the amount of 10 to 30% by weigh of the total weight of the compositions of the invention.

The pharmaceutical vehicles of the invention for drug delivery are an improvement over those prior art methods of drug delivery of the prior art in that the compositions are, preferably, optimized for tolerance in the eye, on the skin, in a body cavity, or for injection, preferably, by formulating the drug delivery compositions so as to have iso-osmotic (isotonic) characteristics in the gel state. Generally, the compositions of the invention also can be formulated to be hyper-isotonic or hypo-isotonic (hyper-osmotic or hypo-osmotic) in the gel state. By matching the osmolality of the drug delivery compositions of the invention, for instance, to those of bodily fluids such as the lacrimal fluid of the eye, it is possible to eliminate burning or other discomfort upon application of the drug delivery systems of the invention to the eye, to the skin, in a body cavity, or by injection. Drugs or diagnostic agents which can be administered to the eye of a mammal by means of the compositions according to the invention are, for example:
antibacterial substances such as beta-lactam antibiotics, such as cefoxitin, n-formamidoylthienamycin and other thienamycin derivatives, tetracyclines, chloramphenicol, neomycin, carbenicillin, colistin, penicillin G, polymyxin B, vancomycin, cefazolin, cephaloridine, chibrorifamycin, gramicidin, bactitracin and sulfonamides;
aminoglycoside antibiotics such as gentamycin, kanamycin, amikacin, sisomicin and tobramycin;
nalidixic acid and its analogs such as norfloxacin and the antimicrobial combination fluoroalanine/pentizidone, nitrofurazones and analogs thereof;
antinistaminics and decongestants such as pyrilamine, chlorpheniramine, tetrahydrazoline, antazoline and analogs thereof; mast-cell inhibitors of histamine release, such as cromolyn;
anti-inflammatories such as cortisone, hydrocortisone, hydrocortisone acetate, betamethasone, dexamethasone, dexamethasone sodium phosphate, prednisone, methylprednisolone, medrysone, fluorometholone, prednisolone, prednisolone sodium phosphate, triamcinolone, indomethacin, sulindac, its salts and its corresponding sulfides, and analogs thereof;
miotics and anticholinergics such as echothiophate, pilocarpine, physostigmine salicylate, diisopropylfluorophosphate, epinephrine, dipivalopylepinephrine, neostigmine, echothiopate iodide, demecarium bromide, carbamoyl choline chloride, methacholine, bethanechol, and analogs thereof;
mydriatics such as atrophine, homatropine, scopolamine, hydroxyamphetamine, ephedrine, cocaine, tropicamide, phenylephrine, cyclopentolate, oxyphenonium, eucatropine, and analogs thereof;

Other drugs can be used in the treatment of conditions and lesions of the eyes such as:
antiglaucoma drugs, for example, timolol, and especially its maleic salt and R-timolol and a combination of timolol or R-timolol with pilocarpine, as well as many other adrenergic agonists and/or antagonists: epinephrine and an epinephrine complex, or prodrugs such as bitartrate, borate, hydrochloride and dipivefrine derivatives; carbonic anhydrase inhibitors such as acetazolamide, dichlorphenamide, 2-(p-hydroxyphenyl)- thio thiophenesulfonamide, 6-hydroxy-2-benzothiazolesulfonamide, and 6-pivaloyloxy-2-benzothiazolesulfonamide;
antiparasitic compounds and/or anti-protozoal compounds such as ivermectin, pyrimethamine, trisulfapidimidine, clindamycin and corticosteroid preparations;
compounds having antiviral activity such as acyclovir, 5-iodo-2'-deoxyuridine (IDU), adenosine arabinoside (Ara-A), trifluorothymidine, interferon, and interferon-inducing agents such as poly I:C;
antifungal agents such as amphotericin B, nystatin, flucytosine, natamycin and miconazole;
anesthetic agents such as etidocaine cocaine, benoxinate, dibucaine hydrochloride, dyclonine hydrochloride, naepaine, phenacaine hydrochloride, piperocaine, proparacaine hydrochloride, tetracaine hydrochloride, hexylcaine, bupivacaine, lidocaine, mepivacaine and prilocaine;
ophthalmic diagnostic agents, such as:
   (a) those used to examine the retina such as sodium fluorescein;
   (b) those used to examine the conjunctiva, cornea and lacrimal apparatus, such as fluorescein and rose bengal; and
   (c) those used to examine abnormal pupillary responses such as methacholine, cocaine, adrenaline, atropine, hydroxyamphetamine and pilocarpine;
ophthalmic agents used as adjuncts in surgery such as alpha-chymotrypsin and hyaluronidase;
chelating agents such as ethylenediaminetetraacetic acid (EDTA) and deferoxamine;
immunosuppressants and anti-metabolites such as methotrexate, cyclophosphamide, 6-mercaptopurine and azathioprine and combinations of the compounds mentioned above, such as antibiotics/antiinflammatories combinations such as the combination of neomycin sulfate and dexamethasone sodium phosphate and combinations concomitantly used for treating glaucoma, for example, a combination of timolol maleate and aceclidine.

With specific reference to the use of the pharmaceutical compositions of the invention for administration to the skin of a mammal, it is contemplated to use suitable medicaments such as antibacterial substances, anti-infectives, anesthetics, anti-inflammatories, anti-parasitics, antivirals, antifungals, analgesics, and diagnostics. Representative antibacterial substances are the antibacterial substances selected from the group consisting of beta-lactam antibiotics, tetracyclines, chloramphenicol, neomycin, gramicidin, bacitracin, sulfonamides, animoglysocide antibiotics, tobramycin, nitrofurazone, nalidixic acid and analogs, the antimicrobial combination of fludalanine/pentizdone, mafenide acetate, silver sulfadiazine, and nitrofurazone. Representative beta-lactam antibiotics or representative anti-infectives are iodine, chloramines, benzalkonium chloride and phenol. Representative anti-inflammatory drugs are cortisone, hydrocortisone, betamethasone, dexamethasone, fluocortolone, prednisolone, triamcinalone, indomethacine, sulindac and its salts and corresponding sulfide. A representative antiparasitic drug is ivermectin. Representative antiviral drugs are acyclovir and interferon. Representative anesthetic drugs are benzocaine, lidocaine and dibucaine. Representative antifungal drugs are tolnaftate, undecylenic acid, salicylic acid, zinc undecylenate miconazole, and thiabendazole. Representative analgesic drugs are methylsalicylate, menthol, camphor, methylnicotinate, triethanolamine salicylate, glycol salicylate and salicylamine. Representative diagnostic compounds are n-alkyl carbonates, cholesteryl oleyl carbonate, cholesteryl nonanoate or cholesteryl benzoate all in proper proportions to effect liquid crystal responses.

With respect to the use of tne pharmaceutical compositions of the invention for injection either subcutaneously or intramuscularly, the following classes of drugs selected from the group consisting of antibacterial substances, antihistamines and decongestants, anti-inflammatories, antiparasitics, antivirals, local anesthetics, antifungal, amoebicidal, or trichomonocidal agents, analgesics, antiarthritics, antiasthmatics, anticoagulants, anticonvulsants, antidepressants, antidiabetics, antineoplastics, antipsychotics, antihypertensives and muscle relaxants. Representative antibacterial substances are beta-lactam antibiotics, tetracyclines, chloramphenicol, neomycin, gramicidin, bacitracin, sulfonamides, aminoglycoside antibiotics, tobramycin, nitrofurazone, nalidixic acid and analogs and the antimicrobial combination of fludalanine/pentizidone. Representative antihistaminics and decongestants are perilamine, chlorpheniramine, tetrahydrozoline and antazoline. Representative antiinflammatory drugs are cortisone, hydrocortisone, betamethasone, dexamethasone, fluocortolone, prednisolone, triamcinolone, indomethacin, sulindac and its salts and corresponding sulfide. A representative antiparasitic compound is ivermectin. Representative antiviral compounds are acyclovir and interferon. Representative analgesic drugs are diflunisal, aspirin or acetaminophen. Representative antiarthritics are phenylbutazone, indomethacin, sulindac, its salts and corresponding sulfide, dexamethasone, ibuprofen, allopurinol, oxyphenbutazone or probenecid. Representative antiasthma drugs are theophylline, ephedrine, beclomethasone dipropionate and epinephrine. Representative anticoagulants are heparin, bishydroxycoumarin, and warfarin. Representative anticonvulsants are diphenylhydrantoin and diazepam. Representative antidepressants are amitriptyline, chlordiazepoxide perphenazine, protriptyline, imipramine and doxepin. Representative antidiabetics are insulin, somatostatin and its analogs, tolbutamide, tolazamide, acetohexamide and chlorpropamide. Representative antineoplastics are adriamycin, fluorouracil, methotrexate and asparaginase. Representative antipsychotics are prochlorperazine, lithium carbonate, lithium citrate, thioridazine, molindone, fluphenazine, trifluoperazine, perphenazine, amitriptyline and triflupromazine. Representative antihypertensives are spironolactone, methyldopa, hydralazine, clonidine, chlorothiazide, deserpidine, timolol, propranolol, metoprolol, prazosin hydrochloride and reserpine. Representative muscle relaxants are succinylcholine-chloride, danbrolene, cyclobenzaprine, methocarbamol and diazepam.

Many pharmaceutically active materials may be delivered to body cavities by the drug delivery system of this invention. Preferably, the drug, or pharmaceutical, is water soluble. Some drugs will show greater solubility in the aqueous polymer system than others. Cosolvents can be used to enhance drug solubility, however, some drugs may be insoluble. These can often be suspended in the polymer vehicle with the aid of suitable suspending or viscosity-enhancing agents.

Suitable classes of drugs which can be administered to a body cavity by the drug polymer delivery system of the present invention are antibacterial substances such as B-lactam antibiotics, such as cefoxitin, n-formamidoyl thienamycin and other thienamycin derivatives, tetracyclines, chloramphenicol, neomycin, gramicidin, bacitracin, sulfonamides; aminoglycoside antibiotics such as gentamycin, kanamycin, amikacin, sisomicin and tobramycin; nalidixic acids and analogs such as norfloxacin and the antimicrobial combination of fludalanine/pentizidone; and nitroflurazones antihistaminics and decongestants such as pyrilamine, cholpheniramine, tetrahydrazoline and antazoline, anti-inflammatories such as cortisone, hydrocortisone, beta-methasone, dexamethasone, fluocortolone, prednisolone, triamcinolone, indomethacin sulindac, its salts and its corresponding sulfide,

Also included are antiparasitic compounds such as ivermectin; antiviral effective compounds such as acyclovir and interferon,

For treatment of vaginal and urethral conditions requiring antifungal, amoebicidal, trichomonacidal agents or antiprotozoals, the following agents can be used: polyoxyethylene nonylphenol, alkylaryl sulfonate, oxyquinoline sulfate, miconazole nitrate, sulfanilamide, candicidin, sulfisoxazole, nysatitin, clotrimazole, metronidazole and antiprotozoals such aschloramphenicol, chloroquine, trimethoprim, sulfamethoxazole.

For use rectally the following suitable drugs can be administered by the drug polymer delivery system of the present invention:
(1) Analgesics such as aspirin, acetaminophen, and deflunisal.
(2) Anesthetics such as lidocaine, procaine, benzocaine, and xylocaine.
(3) Antiarthritics such as phenylbutazone, indomethacin, sulindac, dexamethasone, ibuprofen, allopurinol, and oxyphenbutazone probenecid.
(4) Antiasthma drugs such as theophylline, ephedrine, beclomethasone dipropionate, and epinephrine.
(5) Urinary tract disinfectives such as sulfamethoxazole, trimethoprim, nitrofurantoin, and norloxicin.
(6) Anticoagulants such as heparin, bishydroxy coumarin, and warfarin.
(7) Anticonvulsants such as diphenylhydantoin, and diazepam.
(8) Antidepressants such as amitriptyline, chlordiazepoxide, perphenazine, protriptyline, imipramine, and doxepin.
(9) Antidiabetics such as , insulin, tolbutamide, tolazamide, acetohexamide, and chlorpropamide
(10) Antineoplastics such as adriamycin, flurouracil, methotrexate, and asparaginase
(11) Antipsychotics such as prochlorperazine, lithium carbonate, lithium citrate, thioridazine, molindone, fluphenazinp, trifluoperazine, perphenazine, amitriptyline, and triflupromazine.
(12) Antihypertensive such as spironolactone, methyldopa, hydralazine, clonidine, chlorothiazide, deserpidine, timolol, propranolol, metoprolol, prazosin hydrochloride, and reserpine, and
(13) Muscle relaxants such as mephalan, danbrolene, cyclobenzaprine, methocarbamol, and diazepam.
(14) Antiprotozoals such as chloramphenicol, chloroquine, trimethoprim and sulfamethoxazole.
(15) Spermicidals such as nonoxynol-9.

The particular drug used in the pharmaceutical composition of this invention is the type which a patient would require for pharmacological treatment of the condition from which said patient is suffering. For example, if the patient is suffering from pain or itch of the external auditory canal, the drug of choice would probably be benzocaine.

Also included in this invention is the use of the drug delivery device or pharmaceutical composition minus the active drug, diagnostic agent, or medicament for restoration or maintenance of vaginal acidity. All the ratios of components as described above would be satisfactory for this composition. For this use one would administer the vehicle as needed at the desired osmolality and pH.

In general the drug delivery system of the present invention will contain from 0.01% to 60% by weight of the medicament or pharmaceutical, from 10 to 50% of the polymer and from 80% to 20% water. In special situations, however, the amounts may be varied to increase or decrease the dosage schedule.

If desired, the drug delivery compositions, laser ablatable corneal mask, and corneal protective compositions of the invention may also contain preservatives, cosolvents, suspending agents, viscosity enhancing agents, ionic-strength and osmolality adjustors and other excipients in addition to the medicament and buffering agents. Suitable water soluble preservatives which may be employed in the drug delivery vehicle are sodium bisulfite, sodium thiosulfate, ascorbate, benzalkonium chloride, chlorabutanol, thimerosal, phenylmercuric borate, parabens, benzylalcohol phenylethanol and others. These agents may be present, generally, in amounts of 0.001% to 5% by weight and, preferably, in the amount of of 0.01 to 2% by weight.

Representative buffering agents or salts useful in maintaining the pH at about 7.4 + 0.2 are alkali or alkaline earth metal carbonates, chlorides, sulfates, phosphates, bicarbonates, citrates, borates, acetates and succinates such as sodium phosphate, citrate, borate, acetate, bicarbonate, carbonate and tromethamine (TRIS). These agents are present in amounts sufficient to maintain the pH of the system at 7.4 + 0.2 and preferably, 7.4. As such, the buffering agent can be as much as 5% on a weight basis of the total composition.

The corneal mask compositions of the invention are an improvement over the prior art thermally-reversible gels containing a polyoxyalkylene polymer as the sole polymer, in that the compositions of the invention provide greater gel strength because they are more resistant to shear thinning and are characterized as thermally-irreversible. These advantages are obtained by the incorporation of an ionic polysaccharide in admixture with a water soluble film forming polymer. They can be optimized for optimum physiological tolerance in the eye by formulating the compositions so as to have a neutral pH and isotonic characteristics. These former advantages are obtained by the incorporation of an ionic polysaccharide in admixture with a water soluble, film forming polymer. By matching the osmolality and pH of the laser ablatable corneal mask compositions of the invention to those of the lacrimal fluid of the eye, it is possible to eliminate burning or other discomfort upon application of the corneal mask of the invention to the eye. The higher gel strength compositions upon contact with a counter-ion allow retention of the gel as an in situ formed corneal mask for long intervals.

The preparation of the compositions of the invention is described below. The Examples which follow were prepared according to the following preparation procedure. Since the polymer systems of this invention dissolve more completely at reduced temperatures, the preferred method of solubilization (cold process) is to start by adding the required amount of polymer to the amount of cold water to be used. Generally after wetting the polymer by shaking, the mixture is capped and placed in a cold chamber or in a thermostatic container at about 0° C to 10° C in order to dissolve the polymer. The mixture can be stirred or shaken to bring about a more rapid solution of the polymer. The pharmacologically active medicaments and various additives such as buffers, salts, and preservatives can subsequently be added and dissolved. In some instances, the pharmacologically active substance must be suspended since it is insoluble in water.

The following Examples illustrate the various aspects of the invention but are not intended to limit its scope. Where not otherwise specified throughout this specification and claims, temperatures are given in degrees centigrade and parts, percentages, and proportions are by weight.

### EXAMPLE 1

This Example formulation describes a composition of the invention for ophthalmic use as a laser ablatable corneal mask or protective corneal contact lens. The composition prepared was characterized as iso-osmotic, sterile, and having a pH of 7.4 + 0.2. an aqueous solution was made of a polyoxyethylene-polyoxypropylene block copolymer having the structure generically shown above as Formula IV and having a polyoxypropylene hydrophobe base average molecular weight of about 4000, a total average molecular weight of about 11,500, and containing oxyethylene groups in the amount of about 70% by weight of the total weight of copolymer. This copolymer (Formula VI below) is sold under the trademark PLURONIC® F-127 (also known as Poloxamer 407) by the BASF Corporation, Parsippany, New Jersey. A solution in TRIS hydrochloride buffer was made by dissolving said polymer in cold (4° C) buffer to give a concentration of 25% by weight in accordance with the cold process procedure described above for forming aqueous solutions. More specific solution procedures are described in "Artificial Skin I Preparation and Properties of PLURONIC F-127 Gels For Treatment of Burns". Journal of Biomedical Material Research 6, 527, 1972,
The block copolymer has the formula:

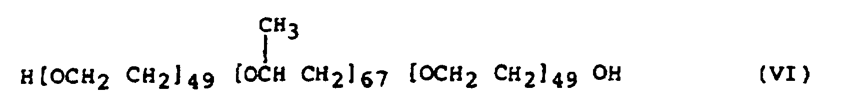

This formulation forms the basis for the Figure in which the curve shows the penetration of a 20mm thickness aqueous gel at various temperatures. After contact of the gel with calcium ions, as indicated by the vertical line at 40°C, the gel strength is not reduced or the composition rendered fluid by lowering the temperature to 25°C.

The formulation was sterilized by autoclaving at 121°C and 103.4x10³ Nm⁻² (15 psi) for 15 minutes. The pH before autoclaving was found co be 7.3 and after autoclaving remained the same. The osmolality in the gelled state before autoclaving was determined to be 290 + 10 and after autoclaving 298 + 10 mOsm/Kg. The gel strength (viscosity) in mPas (centipoise), as measured at 37 degrees °C using a Brookfield (spindle and cup) viscometer at 20 revolutions per minute, was treater than 44,000 before autoclaving and greater than 44,000 after autoclaving.

### EXAMPLE 2 (Inventive) and EXAMPLE 3 (Control)

These examples describe hyperosmotic, pH balanced, thermo-sensitive drug delivery systems, in which the active drug is dissolved. The following antibacterial formulations were prepared to contain 11.2 percent by weight of mafenide acetate. The antibacterial formulations were prepared as follows:

| | Example 2 | Example 3 (Control) |
|---|---|---|
| Ingredient | Percent by Weight | |
| Mafenide acetate | 11.2 | 11.2 |
| Sodium alginate | 0.5 | -- |
| Carrageenan | -- | 0.57 |
| Poloxamer 407 (BASF) | 19.0 | 19.0 |
| TRIS hydrochloride buffer (0.1 molar) | 69.3 | 69.3 |

The formulations were prepared by dissolving the drug and sodium alginate or carrageenan by stirring in the required amount of TRIS hydrochloride buffer. These ingredients were placed in a glass beaker in an ice bath and the Poloxamer 407 was added to the beaker slowly while stirring. After the Poloxamer 407 was completely dissolved, the formulation was stored at 4°C. The entire process was carried out under a nitrogen atmosphere. The product obtained was characterized as clear, straw colored and exhibiting gelation at the temperature of mammalian skin (33±2°C.). In the gelled state, the pH and osmolality of the preparation would be expected to be 7.5 and over 720 mOsm/Kg, respectively. Iso-osmotic solutions containing 2.5 to 3 percent by weight of the drug would be expected to be iso-osmotic but less therapeutically effective. The solutions of Examples 2 and 3 were exposed to an equal amount of a 2% by weight solution of calcium chloride. The solution of Example 2 formed a thermo-irreversible gel. The solution of Example 3 remained thermo-reversible.

Comparison of these examples illustrates the importance of utilizing an ionic polysaccharide (sodium alginate) instead of a non-ionic polysaccharide (carrageenan). In these examples, the 2% solution of calcium chloride was used to impregnate a gauze bandage and subsequently the solutions of Examples 2 and 3 were placed in contact with the gauze bandage. In both cases, the solution of Example 2 was rendered thermo-irreversible and the solution of Example 3 was unaffected.

### EXAMPLE 4 (Invention) and EXAMPLE 5 (Control)

Examples 2 and 3 are repeated substituting for poloxamer 407, 2% by weight of polymer #2, as described in U.S. 4,810,503 and 4% by weight of surfactant #1, as described therein. The balance of the percentage of Poloxamer 407 used in Examples 2 and 3 is made up with TRIS hydrochloride buffer. These formulations form soft gels at room temperature which are usefully stiffened upon exposure to a 2% by weight aqueous solution of calcium chloride, in the case of Example 4, and are unaffected, in the case of control Example 5. Substantially similar pH and osmolality results are obtained.

### EXAMPLE 6

Ion exchange resin beads sold under the tradename Duolite were treated so as to incorporate calcium by first treating a 30 gram sample of the ion exchange resin with a solution of 0.1 molar hydrochloric acid so as to allow for the exchange of protons for sodium. After three washings with 0.1 molar hydrochloric acid, the beads were washed with water and then washed twice with a 2% aqueous solution of calcium chloride. Each of the washing steps took place over a period of 16 hours (overnight). The beads were thereafter filtered and washed with water utilizing coarse filter paper and a Buchner glass filter assembly. The beads were then left overnight in a desiccator to dry. The dried beans of ion exchange resin which were obtained were utilized in the amount of 2 grams to fill a first compartment (close to the needle of the syringe) of a glass syringe utilized to apply liquids and dry materials. The syringe is sold under the tradename Hypak. Into the second compartment of the syringe, there was placed the solution of Example 2. Pushing the plunger of the syringe forward resulted in mixing the solution of Example 2 with the ion exchange beads. After 5 to 10 minutes subsequent to mixing, the mixture was expelled from the syringe. After an additional 15 minutes the expelled material formed a thermo-irreversible film on the substrate on which it was expelled.

### EXAMPLES 7 and 8

These examples describe pH balanced, thermo-sensitive aqueous systems which are suitable for forming a thermally reversible corneal contact lens or laser ablatable corneal mask in situ. Both examples will result in the formation of thermally-irreversible systems upon exposure to an aqueous solution of 2% to 10% by weight calcium chloride. The formulations are:

| | Example 7 | Example 8 |
|---|---|---|
| Ingredient | Percent by Weight | |
| Poloxamer 407 (block, BHT free) | 16.0 | 16.0 |
| Sodium alginate | 1.0 | 1.0 |
| Boric acid-Borate Buffer | | |
| pH 7.4 | 82.7 | - |
| Phosphate-Borate Buffer | | |
| pH 7.4 | - | 82.7 |
| Glycerin | 0.3 | 0.3 |

The formulations are prepared by the "Hot Method", "BWC surfactants in gel cosmetics", I.R. Schmolka, Cosmetics and Toiletries, vol 92, July 1977, pages 77-79. The procedure is as follows:
1. The poloxamer blocks (BASF Corp) are melted at 65°C in a water jacketed mixing bowl. The mixer used is a Stephan UMC5 mixer-blender (Stephan Machinery, Columbus, OH).
2. A weighed amount of buffer is placed in a one liter beaker. Weighed amounts of Glycerin (JT Baker) and Sodium Alginate (Protonal SF120, Protan, Inc.) are added to dissolve and mix.
3. This solution is added to the molten poloxamer and mixed at 65°C for 15 minutes in a nitrogen atmosphere.
4. The temperature is gradually dropped to 25°C and then to 15°C by the circulation of ice-cold water.
5. The final product is stored overnight at 4°C in a glass beaker.
6. The next day the following tests were done and the results were as follows:

| | Test | Example 7 | Example 8 |
|---|---|---|---|
| 1. | pH | 7.37 | 7.44 |
| 2. | Osmolality in gelled state | 290 | 350 |
| | (calculated m0sm/kg) | iso-osmotic | hyper-osmotic |
| 3. | Solution-Gel Profile | strong gel | weak gel |
| | (Brookfield Viscometer) (10 rpm, at 33°C) | 50,000 mPas(cps) | 1000 mPas (cps) |

### EXAMPLES 9 and 10

Examples 7 and 8 are repeated substituting for poloxamer 407, 2% by weight of polymer #2, as described in U.S. 4,810,503 and 4% by weight of surfactant #1, as described therein. The balance of the percentage of Poloxamer 407 used in Examples 7 and 8 is made up with borate or phosphate-borate buffer, respectively. These formulations form soft gels at room temperature which are usefully stiffened upon exposure to a 2% to 10% by weight aqueous solution of calcium chloride. Substantially similar pH and osmolality results are obtained.

### EXAMPLE 11

This example describes the application of the solution of Example 7 to the cornea of a rabbit eye and the conversion of a thermally-reversible gel to a thermally-irreversible gel by the application of a 10% calcium chloride solution having a pH of 6.9. The calcium chloride solution is applied to the concave surface of a contact lens prior to contacting the surface of the gel formed upon the cornea of the rabbit eye utilizing the composition of Example 7. After applying the composition of Example 7 to the cornea of a rabbit placed under general anesthesia, it is found that a thermally-reversible gel forms upon contact with the cornea. Subsequently, a 10% aqueous solution of calcium chloride is applied to the concave surface of a hard contact lens and the contact lens is placed over the thermally-reversible gel coating the cornea of the rabbit eye. The time required for a thermally-reversible gel to convert to a thermally-irreversible gel is approximately 5 minutes. Thereafter, the contact lens is removed to expose a perfectly smooth and optically clear gelled surface of the composition of Example 7. Excimer laser keratectomy is thereafter performed utilizing an argon fluoride excimer laser (193 nm). Further details of excimer laser keratectomy process can be found in Archives of Ophthamology, Vol. 106, Feb., 1988, entitled "Excimer Laser Kecatectomy for Myopia with a Rotating-slit Delivery System", Hanna et al.

## Claims

1. A pH buffered thermally-reversible, aqueous gel composition **characterized in that** it comprises a polyoxyalkylene block copolymer and an alginate or chitosan ionic polysaccharide.

2. A composition as claimed in claim 1, which further comprises a counter-ion in latent form.

3. A composition as claimed in claim 1 or 2, wherein said polyoxyalkylene block copolymer has the formula:-
Y[(A)ₙ―E―H]ₓ (I)
wherein: A is a polyoxyalkylene moiety having an oxygen/carbon atom ratio of less than 0.5, x is at least 2, Y is derived from water or an organic compound containing x reactive hydrogen atoms, E is a polyoxyethylene moiety constituting at least 60% by weight of the copolymer, n has a value such that the average molecular weight is at least 500 to 900, as determined by the hydroxyl number of an intermediate,
Y[(A)ₙ―H]ₓ (II)
and the total average molecular weight of the copolymer is at least about 5000.

4. A composition as claimed in claim 3, wherein said copolymer has the formula:
HO(C₂H₄O_{)b}(C₄H₈O)ₐ(C₂H₄O)_{b}H (III)
wherein in III, a is an integer such that the hydrophobe base represented by (C₄H₈O)ₐ has a molecular weight of at least about 500 as determined by hydroxyl number, the polyoxyalkylene chain constitutes at least about 70% by weight of the copolymer, and the copolymer has a total average molecular weight of at least 15,000; or
HO(C₂H₄O)_{b}(C₃H₆O)ₐ(C₂H₄O)_{b}H (IV)
wherein in IV, a is an integer such that the hydrophobe base represented by (C₃H₆O)ₐ has a molecular weight of at least about 900 average molecular weight as determined by hydroxyl number, the polyoxyethylene chain constitutes at least about 70% by weight of the copolymer, and the copolymer has a total average molecular weight of at least about 15,000; or 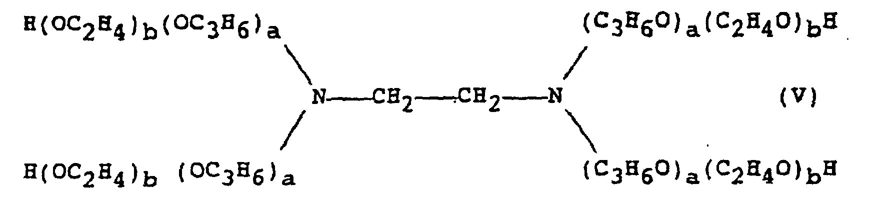 wherein in V, a and b are integers such that the copolymer has a hydrophobe molecular weight of at least about 1500, a hydrophile content of at least about 70%, and a total average molecular weight of at least about 15,000 and wherein said polyoxyalkylene block copolymer is present in the amount of about 10% to about 50% by weight.

5. A composition as claimed in Claim 3, wherein said polyoxyalkylene block copolymer is, 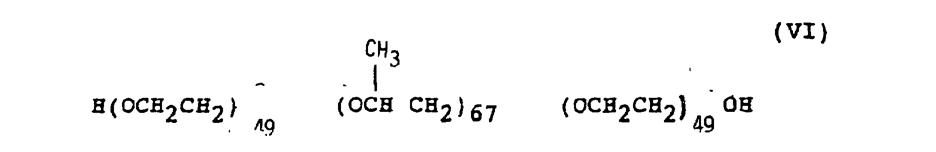 present in the amount of about 10 to about 30% by weight of the total weight of said composition.

6. A pH buffered aqueous gel composition **characterised in that** it comprises a polyoxyalkylene polyether having a molecular weight of from 10,000, a surfactant and the product formed by the contacting of an alginate or chitosan ionic polysaccharide with a counter-ion.

7. A composition as claimed in claim 6, wherein said polyether is either:
(A) a polyoxyalkylene polyether prepared by reacting ethylene oxide and at least one lower alkylene oxide having 3 to 4 carbon atoms with at least one active hydrogen-containing compound having from 3 to 10 carbon atoms and from 3 to 6 active hydrogens to prepare a heteric or block copolymer intermediate and further reacting said copolymer intermediate with at least one alpha-olefin oxide having an average carbon chain length of about 20 to about 45 aliphatic carbon atoms and wherein said alpholefin oxide is present in the amount of about 0.3 to 10 percent by weight based upon the total weight of said polyether; or
(B) a polyoxyalkylene polyether prepared by reacting ethylene oxide with at least one active hydrogen-containing compound having from 2 to 10 carbon atoms and from 2 to 6 active hydrogens to prepare a homopolymer intermediate and further reacting said homopolymer with at least one alpha-olefin oxide having an average carbon chain length of about 20 to 45 aliphatic carbon atoms and wherein said alpha-olefin oxide is present in the amount of about 0.3 to 10 percent by weight based on the total weight of said polyether.

8. A composition as claimed in any of claims 3 to 7 which further comprises a drug or diagnostic agent and in which said counter-ion is a divalent or trivalent metal counter-ion.

9. A protective corneal contact lens or an ablatable corneal mask comprising a composition as claimed in any of claims 1 to 8.

10. A delivery vehicle for a drug or a diagnostic agent comprising a composition as claimed in any of claims 1 to 7.

11. A composition as claimed in any of claims 1 to 7 for use in forming in situ a protective corneal contact lens or an ablatable corneal mask.

12. A composition as claimed in any of claims 1 to 7 for use in the treatment of corneal astigmatism, myopia and/or hyperopia.

13. A composition as claimed in any of claims 1 to 7 for delivering a drug or diagnostic agent to a mammal.

14. The use of a composition as claimed in any of claims 1 to 7 for the manufacture of a medicament for forming in situ a protective corneal contact lens or an ablatable corneal mask.

15. The use of a composition as claimed in any of claims 1 to 7 for the manufacture of a medicament for delivering a drug or diagnostic agent to a mammal.

## Patentansprüche

1. pH-Gepufferte, thermisch reversible, wässrige Gelzusammensetzung, **dadurch gekennzeichnet, dass** sie ein Polyoxyalkylen-Blockcopolymer und ein ionisches Alginatoder Chitosan-Polysaccharid umfasst.

2. Zusammensetzung nach Anspruch 1, die ferner ein Gegenion in latenter Form umfasst.

3. Zusammensetzung nach Anspruch 1 oder 2, worin genanntes Polyoxyalkylen-Blockcopolymer die folgende Formel aufweist:
Y[(A)ₙ―E―H]ₓ (I)
worin: A eine Polyoxyalkylenkomponente mit einem Sauerstoff/Kohlenstoffatom-Verhältnis von weniger als 0,5 ist, x mindestens 2 ist, Y von Wasser oder einer organischen Verbindung hergeleitet wird, die x reaktive Wasserstoffatome enthält, E eine Polyoxyethylenkomponente ist, die mindestens 60 Gew.-% des Copolymers ausmacht, n einen Wert dergestalt aufweist, dass das durchschnittliche Molekulargewicht, wie durch die Hydroxylzahl eines Intermediärproduktes
Y[(A)ₙ―H]ₓ (II)
bestimmt, mindestens 500 bis 900 ist und das durchschnittliche Gesamtmolekulargewicht des Copolymers mindestens ca. 5000 beträgt.

4. Zusammensetzung nach Anspruch 3, worin genanntes Copolymer die folgende Formel aufweist:
HO(C₂H₄O)_{b}(C₄H₈O)ₐ(C₂H₄O)_{b}H (III)
worin in III: a eine ganze Zahl dergestalt ist, dass die hydrophobe Basis, dargestellt durch (C₄H₈O)ₐ, ein Molekulargewicht, wie durch die Hydroxylzahl bestimmt, von mindestens 500 aufweist, die Polyoxyalkylenkette mindestens ca. 70 Gew.-% des Copolymers ausmacht und das Copolymer ein durchschnittliches Gesamtmolekulargewicht von mindestens 15 000 aufweist oder
HO(C₂H₄O)_{b}(C₃H₆O)ₐ(C₂H₄O)_{b}H (IV)
worin in IV: a eine ganze Zahl dergestalt ist, dass die hydrophobe Basis, dargestellt durch (C₃H₆O)ₐ, ein Molekulargewicht, wie durch die Hydroxylzahl bestimmt, von mindestens ca. 900 (durchschnittliches Molekulargewicht) aufweist, die Polyoxyethylenkette mindestens ca. 70 Gew.-% des Copolymers ausmacht und das Copolymer ein durchschnittliches Gesamtmolekulargewicht von mindestens ca. 15 000 aufweist oder 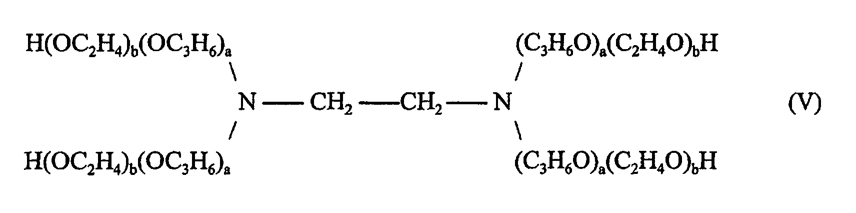 worin in V: a und b ganze Zahlen dergestalt sind, dass das Copolymer ein Molekulargewicht des hydrophoben Anteils von mindestens ca. 1500, einen hydrophilen Gehalt von mindestens ca. 70 % und ein durchschnittliches Gesamtmolekulargewicht von mindestens 15 000 aufweist und worin genanntes Polyoxyalkylen-Blockcopolymer in der Menge von ca. 10 Gew.-% bis ca. 50 Gew.-% vorliegt.

5. Zusammensetzung nach Anspruch 3, worin genanntes Polyoxyalkylen-Blockcopolymer 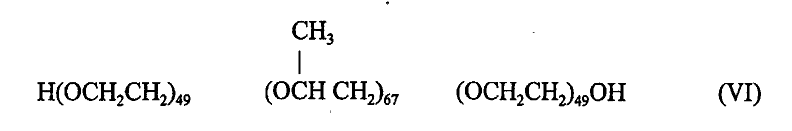 ist, das in der Menge von ca. 10 Gew.-% bis ca. 30 Gew.-% des Gesamtgewichtes von genannter Zusammensetzung vorliegt.

6. pH-Gepufferte wässrige Gelzusammensetzung, **dadurch gekennzeichnet. dass** sie einen Polyxoyalkylenpolyether mit einem Molekulargewicht von 10 000, ein oberflächenaktives Mittel und das durch das Kontaktieren eines ionischen Alginat- oder Chitosan-Polysaccharids mit einem Gegenion gebildete Produkt aufweist.

7. Zusammensetzung nach Anspruch 6, worin genannter Polyether Folgendes ist:
(A) Entweder ein Polyoxyalkylenpolyether, zubereitet durch zur Reaktion bringen von Ethylenoxid und mindestens einem niederen Alkylenoxid mit 3 bis 4 Kohlenstoffatomen mit mindestens einer aktiven, Wasserstoff enthaltenden Verbindung mit von 3 bis 10 Kohlenstoffatomen und von 3 bis 6 aktiven Wasserstoffen zum Zubereiten eines "heterischen" oder Blockcopolymer-Intermediärproduktes und ferner zur Reaktion bringen von genanntem Copolymer-Intermediärprodukt mit mindestens einem α-Olefinxoxid mit einer durchschnittlichen Kohlenstoffkettenlänge von ca. 20 bis ca. 45 aliphatischen Kohlenstoffatomen und worin genanntes α-Olefinoxid in der Menge von ca. 0,3 bis 10 Gew.-% basierend auf dem Gesamtgewicht von genanntem Polyether vorliegt oder
(B) ein Polyoxyalkylenpolyether, zubereitet durch zur Reaktion bringen von Ethylenoxid mit mindestens einer aktiven, Wasserstoff enthaltenden Verbindung mit mindestens von 2 bis 10 Kohlenstoffatomen und von 2 bis 6 aktiven Wasserstoffen zum Zubereiten eines Homopolymer-Intermediärproduktes und ferner zur Reaktion bringen von genanntem Homopolymer mit mindestens einem α-Olefinoxid mit einer durchschnittlichen Kohlenstoffkettenlänge von ca. 20 bis 45 aliphatischen Kohlenstoffatomen und worin genanntes α-Olefinoxid in der Menge von ca. 0,3 bis 10 Gew.-% basierend auf dem Gesamtgewicht von genanntem Polyether vorliegt.

8. Zusammensetzung nach einem der Ansprüche 3 bis 7, die ferner ein Arzneimittel oder Diagnostikum umfasst und worin genanntes Gegenion ein divalentes oder trivalentes Metallgegenion ist.

9. Protektive Korneakontaktlinse oder eine ablatierbare komeale Maske, die eine Zusammensetzung nach einem von Ansprüchen 1 bis 8 umfasst.

10. Ein Abgabeträger für ein Arzneimittel oder ein Diagnostikum, das eine Zusammensetzung nach einem der Ansprüche 1 bis 7 umfasst.

11. Zusammensetzung nach einem der Ansprüche 1 bis 7 zur Verwendung beim Bilden einer protektiven Korneakontaktlinse oder einer ablatierbaren kornealen Maske in situ.

12. Zusammensetzung nach einem der Ansprüche 1 bis 7 zur Verwendung bei der Behandlung von kornealem Astigmatismus, Myopie und/oder Hyperopie.

13. Zusammensetzung nach einem der Ansprüche 1 bis 7 zur Abgabe eines Arzneimittels oder Diagnostikums an ein Säugetier.

14. Verwendung einer Zusammensetzung nach einem der Ansprüche 1 bis 7 für die Herstellung eines Medikaments zum Bilden einer protektiven Korneakontaktlinse oder einer ablatierbaren kornealen Maske.

15. Verwendung einer Zusammensetzung nach einem der Ansprüche 1 bis 7 für die Herstellung eines Medikamentes zur Abgabe eines Arzneimittels oder Diagnostikums an ein Säugetier.

## Revendications

1. Composition de gel aqueux thermiquement réversible et de pH tamponné, **caractérisée en ce qu'**elle comprend un copolymère bloc de polyoxyalkylène et un polysaccharide ionique de chitosane ou d'alginate.

2. Composition selon la revendication 1, comprenant en outre un contre-ion sous forme latente.

3. Composition selon la revendication 1 ou 2, dans laquelle ledit copolymère bloc de polyoxyalkylène a la formule :
Y[(A)ₙ―E―H]ₓ (I)
dans laquelle A représente un motif de polyoxyalkylène ayant un rapport d'atomes oxygène/carbone inférieur à 0,5, x vaut au moins 2, Y est dérivé de l'eau ou d'un composé organique contenant x atomes d'hydrogène réactifs, E représente un motif de polyoxyéthylène constituant au moins 60% en poids du copolymère, n a une valeur telle que le poids moléculaire moyen est d'au moins 500 à 900, tel que déterminé par l'indice d'hydroxyle d'un intermédiaire,
Y[(A)ₙ―H]ₓ (II)
et le poids moléculaire moyen total du copolymère est d'au moins environ 5 000.

4. Composition selon la revendication 3, dans laquelle ledit copolymère a la formule :
HO(C₂H₄O)_{b}(C₄H₈O)ₐ(C₂H₄O)_{b}H (III)
dans laquelle dans III, a représente un nombre entier tel que la base hydrophobe représentée par (C₄H₈O)ₐ a un poids moléculaire d'au moins environ 500, tel que déterminé par l'indice d'hydroxyle, la chaîne de polyoxyalkylène constitue au moins environ 70% en poids du copolymère, et le copolymère a un poids moléculaire moyen total d'au moins 15 000 ; ou
HO(C₂H₄O)_{b}(C₃H₆O)ₐ(C₂H₄O)_{b}H (IV)
dans laquelle dans IV, a représente un nombre entier tel que la base hydrophobe représentée par (C₃H₆O)ₐ a un poids moléculaire d'au moins environ 900, poids moléculaire moyen tel que déterminé par l'indice d'hydroxyle, la chaîne de polyoxyéthylène constitue au moins environ 70% en poids du copolymère, et le copolymère a un poids moléculaire moyen total d'au moins environ 15 000 ; ou 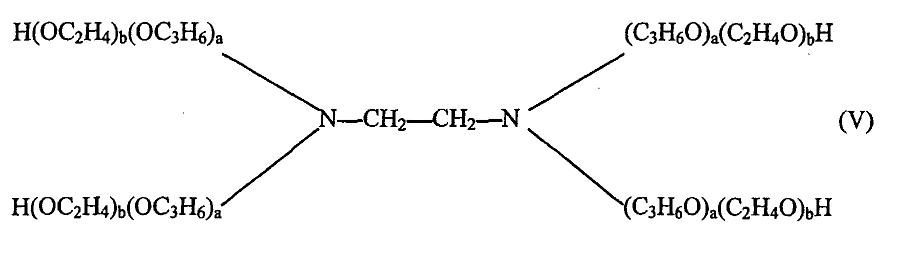 dans laquelle dans V, a et b représentent des nombres entiers tels que le copolymère a un poids moléculaire hydrophobe d'au moins environ 1 500, une teneur hydrophile d'au moins environ 70%, et un poids moléculaire moyen total d'au moins environ 15 000, et dans laquelle ledit copolymère bloc de polyoxyalkylène est présent en une quantité d'environ 10% à environ 50% en poids.

5. Composition selon la revendication 3, dans laquelle ledit copolymère bloc de polyoxyalkylène est 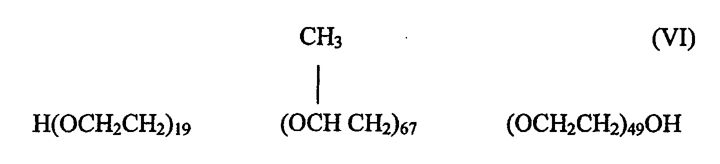 présent en une quantité d'environ 10 à environ 30% en poids du poids total de ladite composition.

6. Composition de gel aqueux de pH tamponné, **caractérisée en ce qu'**elle comprend un polyéther de polyoxyalkylène ayant un poids moléculaire à partir de 10 000, un agent tensioactif et le produit formé par la mise en contact d'un polysaccharide ionique de chitosane ou d'alginate avec un contre-ion.

7. Composition selon la revendication 6, dans laquelle ledit polyéther est soit:
(A) un polyéther de polyoxyalkylène préparé par la réaction d'oxyde d'éthylène et au moins un oxyde d'alkylène inférieur ayant de 3 à 4 atomes de carbone, avec au moins un composé à hydrogène actif, ayant de 3 à 10 atomes de carbone et de 3 à 6 hydrogènes actifs, pour préparer un intermédiaire de copolymère bloc ou hétérique, et la réaction ultérieure dudit intermédiaire de copolymère avec au moins un oxyde d'alpha-oléfine ayant une longueur de chaîne carbonée moyenne d'environ 20 à environ 45 atomes de carbone aliphatiques, et dans laquelle ledit oxyde d'alpha-oléfine est présent en une quantité d'environ 0,3 à 10% en poids sur la base du poids total dudit polyéther ; soit
(B) un polyéther de polyoxyalkylène préparé par la réaction d'oxyde d'éthylène avec au moins un composé à hydrogène actif, ayant de 2 à 10 atomes de carbone et de 2 à 6 hydrogènes actifs, pour préparer un intermédiaire d'homopolymère et la réaction ultérieure dudit homopolymère avec au moins un oxyde d'alpha-oléfine ayant une longueur de chaîne carbonée moyenne d'environ 20 à 45 atomes de carbone aliphatiques, et dans laquelle ledit oxyde d'alpha-oléfine est présent en une quantité d'environ 0,3 à 10% en poids sur la base du poids total dudit polyéther.

8. Composition selon l'une quelconque des revendications 3 à 7, comprenant en outre un médicament ou un agent de diagnostic et dans laquelle ledit contre-ion est un contre-ion métallique divalent ou trivalent.

9. Lentille de contact cornéenne protectrice ou masque cornéen pouvant subir une ablation, comprenant une composition selon l'une quelconque des revendications 1 à 8.

10. Véhicule d'administration d'un médicament ou d'un agent de diagnostic, comprenant une composition selon l'une quelconque des revendications 1 à 7.

11. Composition selon l'une quelconque des revendications 1 à 7, pour une utilisation dans la formation in situ d'une lentille de contact cornéenne protectrice ou d'un masque cornéen pouvant subir une ablation.

12. Composition selon l'une quelconque des revendications 1 à 7, pour une utilisation dans le traitement de l'astigmatisme cornéen, de la myopie et/ou de l'hypermétropie.

13. Composition selon l'une quelconque des revendications 1 à 7, pour l'administration d'un médicament ou d'un agent de diagnostic à un mammifère.

14. Utilisation d'une composition selon l'une quelconque des revendications 1 à 7, pour la fabrication d'un médicament pour la formation in situ d'une lentille de contact cornéenne protectrice ou d'un masque cornéen pouvant subir une ablation.

15. Utilisation d'une composition selon l'une quelconque des revendications 1 à 7, pour la fabrication d'un remède pour l'administration d'un médicament ou d'un agent de diagnostic à un mammifère.
